# FASCICULE DE BREVET EUROPEEN

(11) **EP 3 479 366 B1**
(45) Date de publication et mention de la délivrance du brevet: **22.04.2020**
(21) Numéro de dépôt: 17732943.0
(22) Date de dépôt: 28.06.2017
(51) Int. Cl.: G08B 13/08

(54) **PROCÉDÉ DE SURVEILLANCE DE L'OUVERTURE D'UN CONTENANT**
VERFAHREN ZUR ÜBERWACHUNG DER ÖFFNUNG EINES BEHÄLTERS
METHOD FOR MONITORING THE OPENING OF A CONTAINER

(30) Priorité: 30.06.2016 FR 1656251
(43) Date de publication de la demande: 08.05.2019
(73) Titulaire: Safran Electronics & Defense, 92100 Boulogne-Billancourt (FR)
(72) Inventeur: COUTURIER, Emmanuel, 92100 Boulogne Billancourt (FR); FANTON, Nicolas, 92100 Boulogne Billancourt (FR); TINE, Pierre-Jean, 92100 Boulogne Billancourt (FR)
(74) Mandataire: Lavaud, Thomas
(86) Numéro de dépôt international: PCT/EP2017/066062
(87) Numéro de publication internationale: WO 2018/002180

(56) Documents cités:
- US-A1- 2006 055 533
- US-A1- 2015 039 267
- US-B1- 7 061 377

## Description

L'invention concerne le domaine de la surveillance de l'ouverture de contenants.

### ARRIERE PLAN DE L'INVENTION

La plupart des produits industriels ou des marchandises de valeur sont, à un moment où à un autre, stockés dans un contenant quelconque pour leur transport, leur stockage, leur consignation.

Par « contenant », on entend ici tous types de caisses, de conteneurs, de châssis, d'espaces de stockage quelconques (par exemple, un volume libre dans un aéronef, dans un navire, dans un camion, dans un wagon, etc.), délimité par une enceinte munie de moyens d'accès pouvant être ouverts ou fermés (par exemple, un couvercle, une porte, une trappe, etc.).

Il est bien sûr fondamental d'être en mesure de surveiller l'ouverture d'un tel contenant, notamment pour éviter ou détecter la survenue d'un vol ou d'une manœuvre frauduleuse, ou bien pour éviter ou détecter une anomalie de fonctionnement des moyens d'accès du contenant.

Certains contenants comportent à cet effet une tige métallique numérotée (ou « plomb »). Lors du transport d'un contenant, le plomb est mis en place préalablement au départ du contenant de manière à empêcher l'ouverture du contenant. On vérifie à la réception du contenant que le numéro du plomb correspond au numéro du plomb mis en place préalablement au départ du contenant, et on sectionne le plomb pour mettre fin au transport du contenant et permettre l'accès à l'objet stocké dans le contenant. Cette solution est fiable et peu coûteuse, mais est essentiellement manuelle. En outre, lorsque qu'après ouverture, seule une partie du contenu du contenant a été prélevée et qu'on souhaite prévenir un vol de la partie restante, le contenant doit être refermé et un nouveau plomb installé.

Certains contenants sont donc équipés de dispositifs de surveillance électriques. Les dispositifs de surveillance électriques sont soit reliés par une connexion filaire à une source d'énergie externe (le secteur par exemple), ce qui complexifie leur utilisation, soit eux-mêmes autonomes énergétiquement.

Cette dernière solution est complexe à mettre en œuvre, car il convient alors d'assurer une surveillance efficace tout en limitant la consommation électrique des dispositifs de surveillance électriques pour que ceux-ci soient autonomes pendant toute la durée du transport.

Le document US7061377 divulgue un procédé de surveillance de l'ouverture d'une boîte aux lettres comportant des moyens de détection d'ouverture et de fermeture associés à un module de communication propre à transmettre des messages à intervalles réguliers en cas d'ouverture.

### OBJET DE L'INVENTION

L'invention a pour objet de surveiller l'ouverture d'un contenant grâce un dispositif de surveillance ayant une consommation électrique réduite.

### RESUME DE L'INVENTION

En vue de la réalisation de ce but, on propose un procédé de surveillance de l'ouverture d'un contenant, le procédé de surveillance étant mis en œuvre par un dispositif de surveillance comprenant des moyens de détection de l'ouverture et de la fermeture du contenant et un module de communication, le procédé de surveillance comprenant les étapes de :
- mettre le dispositif de surveillance dans un mode de fonctionnement de fermeture lorsque les moyens de détection détectent que le contenant est fermé, mode dans lequel le module de communication envoie à une première fréquence d'état des messages de fermeture signifiant que les moyens de détection détectent que le contenant est fermé ;
- lorsque le dispositif de surveillance est dans le mode de fonctionnement de fermeture et que les moyens de détection détectent une ouverture du contenant, mettre le dispositif de surveillance dans un mode de fonctionnement d'alerte, dans lequel le module de communication envoie à une deuxième fréquence d'état des messages d'ouverture signifiant que les moyens de détection détectent que le contenant est ouvert ;
- lorsque le dispositif de surveillance est dans le mode de fonctionnement d'alerte et que le module de communication reçoit un message de validation d'ouverture signifiant que l'ouverture du contenant a été validée, mettre le dispositif de surveillance dans un mode de fonctionnement de validation d'ouverture, dans lequel le module de communication envoie à une troisième fréquence d'état des messages d'ouverture signifiant que les moyens de détection détectent que le contenant est ouvert.

Certaines situations nécessitent que la surveillance de l'ouverture du contenant et l'envoi de messages d'ouverture ou de fermeture soient réalisés à une fréquence importante, alors que d'autres situations sont compatibles d'une fréquence moins importante de surveillance et d'envoi des messages (notamment lorsque l'ouverture a été validée par un destinataire final du contenant). Le procédé de surveillance selon l'invention permet d'adapter cette fréquence en fonction de la situation et donc de réduire la consommation électrique du dispositif de surveillance.

L'invention sera mieux comprise à la lumière de la description qui suit de modes de mise en œuvre particuliers non limitatifs de l'invention.

### BREVE DESCRIPTION DES DESSINS

Il sera fait référence aux dessins annexés, parmi lesquels :
- la figure la représente des moyens de détection d'un dispositif de surveillance lorsque le contenant est fermé ;
- la figure 1b représente des moyens de détection d'un dispositif de surveillance lorsque le contenant est ouvert ;

- la figure 2 représente schématiquement le fonctionnement du procédé de surveillance selon un premier mode de réalisation de l'invention ;
- la figure 3 représente schématiquement le fonctionnement du procédé de surveillance selon un deuxième mode de réalisation de l'invention.

### DESCRIPTION DETAILLEE DE L'INVENTION

Le procédé de surveillance selon un premier mode de réalisation de l'invention est ici utilisé pour surveiller l'ouverture d'une porte 1 d'un conteneur 2 contenant un objet et stocké dans un hangar.

Le procédé de surveillance selon l'invention est mis en œuvre par un dispositif de surveillance dont est équipé le conteneur 2 et qui est positionné sur le conteneur 2.

Le dispositif de surveillance comporte des moyens de détection de l'ouverture et de la fermeture de la porte 1 du conteneur 2, un microcontrôleur, une pile, des capteurs supervisant l'état du dispositif de surveillance, une mémoire et un module de communication.

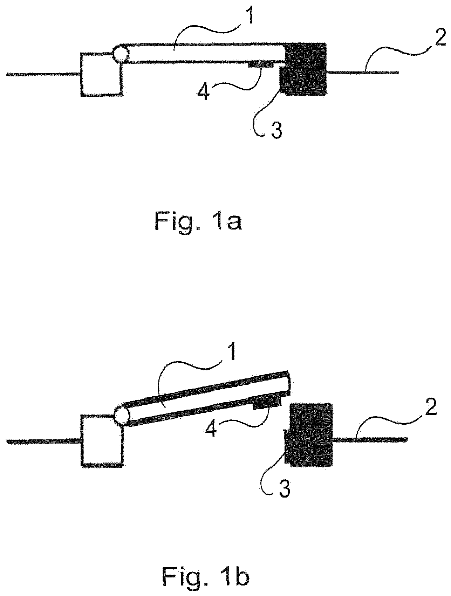

En référence aux figures 1a et 1b, les moyens de détection comprennent un capteur à effet Hall 3 situé sur le conteneur 2. La porte 1 du conteneur 2 comporte un aimant 4 qui génère un champ magnétique.

Lorsque la porte 1 du conteneur 2 est fermée, comme c'est le cas sur la figure la, l'aimant 4 de la porte 1 est positionné à proximité immédiate du capteur à effet Hall 3 qui détecte le champ magnétique généré par l'aimant 4, et qui détecte donc que la porte 1 est fermée.

Lorsque la porte 1 du conteneur 2 est ouverte, comme c'est le cas sur la figure 1b, l'aimant 4 de la porte 1 est éloigné du capteur à effet Hall 3 qui ne détecte pas le champ magnétique généré par l'aimant 4, et qui détecte donc que la porte 1 est ouverte.

Le microcontrôleur, qui est connecté au capteur à effet Hall 3, reçoit donc une information selon laquelle la porte 1 du conteneur 2 (et donc le conteneur 2) est ouverte ou fermée.

Les moyens de détection comportent par ailleurs un premier interrupteur *reed* (ou interrupteur à lame souple) normalement ouvert et un deuxième interrupteur *reed* normalement fermé, situés sur le conteneur 2 à proximité de l'aimant lorsque la porte 1 est fermée. Le premier interrupteur *reed* et le deuxième interrupteur *reed* sont connectés au microcontrôleur.

Le microcontrôleur met ainsi en œuvre un compteur de fermetures, qui s'incrémente à chaque fermeture du premier interrupteur *reed* et donc de la porte 1, et un compteur d'ouvertures, qui s'incrémente à chaque fermeture du deuxième interrupteur *reed* et donc de la porte 1.

Le microcontrôleur détecte une ouverture de la porte 4 lorsque les valeurs du compteur d'ouvertures et du compteur de fermetures sont égales, et une fermeture de la porte 4 lorsque les valeurs du compteur d'ouvertures et du compteur de fermetures sont égales. Le microcontrôleur valide ainsi la détection de l'ouverture ou de la fermeture de la porte 4 réalisée par le capteur à effet Hall.

Le microcontrôleur commande en outre le fonctionnement du dispositif de surveillance.

La pile fournit une énergie électrique pour alimenter de manière exclusive l'ensemble du dispositif de surveillance qui est donc autonome énergétiquement (et qui n'est pas relié par une connexion filaire à une quelconque source d'énergie externe).

Les capteurs supervisant l'état du dispositif de surveillance mesurent des paramètres électriques internes du dispositif de surveillance pour surveiller que celui-ci fonctionne correctement, ainsi que la capacité restante de la pile.

La mémoire permet de stocker un certain nombre de données, et notamment des données d'identification du dispositif de surveillance, du conteneur 2 et de l'objet stocké par le conteneur 2, un certain nombre d'états « ouvert » ou « fermé » du conteneur 2 détectés par le dispositif de surveillance, les paramètres électriques internes, la capacité restante de la pile, la durée d'une ou de plusieurs périodes d'ouverture (au cours desquelles le conteneur 2 reste continuellement ouvert), la durée d'une ou de plusieurs périodes de fermeture (au cours desquelles le conteneur 2 reste continuellement fermé), et divers autres paramètres enregistrés ou bien calculés par le microcontrôleur.

Le module de communication du conteneur 2 comporte une antenne et un émetteur-récepteur radiofréquence. Le module de communication du conteneur 2 forme, avec une unité de communication externe (et, éventuellement, avec des modules de communication d'autres conteneurs stockés à proximité du conteneur 2 évoqué ici), un réseau LPWAN (pour *Low-Power Wide-Area Network,* en anglais) utilisant une technologie conçue pour le domaine de l'internet des objets (ou IoT pour *Internet of Things,* en anglais). La technologie ici utilisée est une technologie de type bande étroite (ou *Narrow Band,* en anglais) ou de type bande ultra étroite (ou UNB de l'anglais *Ultra Narrow Band*) (par exemple LoRa ou SigFox).

Le module de communication possède donc une faible consommation électrique. Le débit des communications mises en œuvre par le module de communication est lui aussi relativement faible, et la taille des messages transmis est relativement réduite (typiquement de douze octets), mais le débit et la taille des messages sont suffisants pour l'application qui nous intéresse ici.

Le module de communication est adapté à communiquer avec l'unité de communication externe de manière bidirectionnelle via le réseau LPWAN. Le module de communication est susceptible d'envoyer tout type de données à l'unité de communication externe, et notamment les données stockées dans la mémoire du dispositif de surveillance.

L'unité de communication externe, qui est située dans le hangar où est stocké le conteneur 2, est quant à elle reliée à un serveur géré ici par le destinataire final de l'objet stocké dans le conteneur 2. Le serveur pourrait bien sûr parfaitement être géré par une personne différente du destinataire final : fabricant de l'objet, responsable de la gestion du hangar, transporteur, gestionnaire de maintenance, etc.

Le destinataire final peut donc être informé à tout moment et n'importe où dans le monde de l'état ouvert ou fermé du conteneur 2 et du bon fonctionnement du dispositif de surveillance (à condition bien sûr que le conteneur 2 soit à une distance de l'unité de communication externe compatible avec le fonctionnement du réseau LPWAN).

On note ici que le dispositif de surveillance se trouve dans un mode de veille la plupart du temps, sauf pendant de courtes durées au cours desquelles il doit effectuer des fonctions de détection, de mesure, d'envoi de messages ou de réception de messages.

Le mode de veille est un mode de fonctionnement à basse consommation d'énergie, dans lequel le module de communication est désactivé tout comme la plupart des composants du dispositif de surveillance (à l'exception, notamment, d'un compteur du microcontrôleur qui gère le réveil du dispositif de surveillance).

On note aussi que les différentes fréquences qui vont être évoquées dans ce qui suit ne correspondent pas à la fréquence utilisée pour communiquer dans le réseau LPWAN (égale par exemple à 2,4GHz), mais à la fréquence à laquelle le dispositif de surveillance sort du mode de veille pour effectuer des fonctions de détection, de mesure, d'envoi de messages ou de réception de messages.

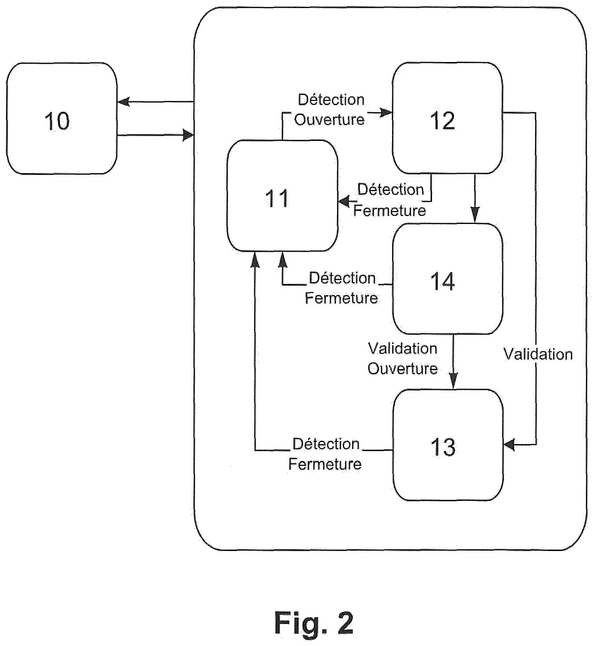

On décrit maintenant, en référence à la figure 2, le fonctionnement du procédé de surveillance selon l'invention.

A la mise sous tension du dispositif de surveillance, le dispositif de surveillance sort du mode de veille 10 et est mis par défaut dans un mode de fonctionnement de fermeture 11 par le microcontrôleur.

Dans le mode de fonctionnement de fermeture 11, les moyens de détection détectent si le conteneur 2 est ouvert ou fermé, les capteurs mesurent les paramètres électriques internes du dispositif de surveillance ainsi que la capacité restante de la pile, et le module de communication envoie à une première fréquence d'état un premier nombre de messages d'état.

La première fréquence d'état est ici égale à un envoi par jour.

Les messages d'état comprennent des messages de fermeture signifiant que les moyens de détection détectent que le conteneur 2 est fermé (on considère que le conteneur 2 est fermé par défaut), ainsi que des informations sur des ouvertures n'ayant pas été suivies de réception de messages d'accusé de réception (on explique plus bas ce que sont les messages d'accusé de réception).

Le module de communication envoie aussi à une première fréquence de supervision un premier nombre de messages de supervision comprenant les paramètres électriques internes du dispositif de surveillance destinés à surveiller que celui-ci fonctionne correctement.

Le module de communication envoie aussi à une fréquence d'autonomie un certain nombre de messages d'autonomie comprenant la capacité restante de la pile ainsi qu'un message d'alerte de fin de vie de la pile si la capacité restante de la pile est inférieure à un seuil de capacité prédéterminé.

Lorsque les moyens de détection détectent que le conteneur 2 est ouvert, le dispositif de surveillance est mis dans un mode de fonctionnement d'alerte 12 par le microcontrôleur.

Dans le mode de fonctionnement d'alerte 12, les moyens de détection détectent si le conteneur 2 est ouvert ou fermé, les capteurs mesurent les paramètres électriques internes du dispositif de surveillance, et le module de communication envoie à une deuxième fréquence d'état un deuxième nombre de messages d'état.

La deuxième fréquence d'état est supérieure à la première fréquence d'état et est ici égale à un envoi toutes les dix secondes.

Le deuxième nombre de messages d'état est supérieur au premier nombre de messages d'état.

Les messages d'état comprennent des messages d'ouverture signifiant que les moyens de détection détectent que le conteneur 2 est ouvert.

Le module de communication envoie aussi à une deuxième fréquence de supervision un deuxième nombre de messages de supervision comprenant les paramètres électriques internes du dispositif de surveillance.

Le module de communication « écoute » par ailleurs le réseau LPWAN en activant la réception de l'émetteur-récepteur à une première fréquence de réception.

Lorsque le dispositif de surveillance se trouve dans le mode de fonctionnement d'alerte 12 et que les moyens de détection détectent que le conteneur 2 est fermé, le dispositif de surveillance est mis dans le mode de fonctionnement de fermeture 11.

Lorsque le dispositif de surveillance se trouve dans le mode de fonctionnement d'alerte 12 et que le module de communication reçoit un message de validation d'ouverture signifiant que l'ouverture du conteneur 2 a été validée par le destinataire final, le dispositif de surveillance est mis dans un mode de fonctionnement de validation d'ouverture 13.

Par « validée par le destinataire final », on entend que le destinataire final indique (via le serveur et l'unité de communication externe) que l'ouverture du conteneur 2 était attendue et ne correspond pas à un dysfonctionnement, à une fraude, à une mauvaise manipulation, etc.

Lorsque le dispositif de surveillance se trouve dans le mode de fonctionnement d'alerte 12 et que le module de communication reçoit un message d'accusé de réception signifiant qu'un message d'ouverture a bien été reçu, le dispositif de surveillance est mis dans un mode de fonctionnement de validation de réception 14. La réception du message d'accusé de réception signifie que l'unité de communication externe reçoit bien les messages (ou au moins une partie des messages) transmis par le module de communication.

Dans le mode de fonctionnement de validation d'ouverture 13, les moyens de détection détectent si le conteneur 2 est ouvert ou fermé, les capteurs mesurent les paramètres électriques internes du dispositif de surveillance, et le module de communication envoie à une troisième fréquence d'état un troisième nombre de messages d'état.

La troisième fréquence d'état est inférieure à la deuxième fréquence d'état et est supérieure à la première fréquence d'état, et est ici égale à un envoi toutes les mille secondes.

Le troisième nombre de messages d'état est inférieur au deuxième nombre de messages d'état et est supérieure au premier nombre de messages d'état.

Les messages d'état comprennent des messages d'ouverture signifiant que les moyens de détection détectent que le conteneur 2 est ouvert.

Le module de communication envoie aussi à une troisième fréquence de supervision un troisième nombre de messages de supervision comprenant les paramètres électriques internes du dispositif de surveillance destinés à surveiller que celui-ci fonctionne correctement.

Si les moyens de détection détectent que le conteneur 2 est fermé, le dispositif de surveillance est mis dans le mode de fonctionnement de fermeture 11.

Dans le mode de fonctionnement de validation de réception 14, les moyens de détection détectent si le conteneur 2 est ouvert ou fermé, les capteurs mesurent les paramètres électriques internes du dispositif de surveillance, et le module de communication envoie à une quatrième fréquence d'état un quatrième nombre de messages d'état.

La quatrième fréquence d'état est inférieure à la deuxième fréquence d'état et est supérieure à la troisième fréquence d'état.

La quatrième fréquence d'état est ici égale à un envoi toutes les cent secondes.

Le quatrième nombre de messages d'état est inférieur au deuxième nombre de messages d'état et est supérieur au troisième nombre de messages d'état.

Les messages d'état comprennent des messages d'ouverture signifiant que les moyens de détection détectent que le conteneur 2 est ouvert.

Le module de communication envoie aussi à une quatrième fréquence de supervision un quatrième nombre de messages de supervision comprenant les paramètres électriques internes du dispositif de surveillance.

Le module de communication « écoute » par ailleurs le réseau LPWAN en activant la réception de l'émetteur-récepteur à une deuxième fréquence de réception.

Si les moyens de détection détectent que le conteneur 2 est fermé, le dispositif de surveillance est mis dans le mode de fonctionnement de fermeture 11.

Si le module de communication reçoit un message de validation d'ouverture signifiant que l'ouverture du conteneur a été validée par le destinataire final, le dispositif de surveillance est mis dans le mode de fonctionnement de validation d'ouverture 13.

Le mode de fonctionnement d'alerte 12, le mode de fonctionnement de validation d'ouverture 13 et le mode de fonctionnement de validation de réception 14 permettent donc d'adapter la fréquence d'état et le nombre de messages d'état envoyés en fonction de la réception des messages d'accusé de réception et de validation d'ouverture.

Dans le mode de fonctionnement d'alerte 12, la deuxième fréquence d'état et le deuxième nombre de messages d'état sont ainsi importants pour maximiser les chances de réception. En effet, dans le mode de fonctionnement d'alerte 12, on ne sait pas si l'ouverture détectée est une ouverture normale ou une ouverture suspecte, et on ne sait même pas si les messages envoyés par le module de communication sont bien reçus.

Une fois qu'un message d'accusé de réception est reçu, la communication avec l'unité de communication externe est établie. Le dispositif de surveillance se trouve alors dans le mode de validation de réception 14, et donc la quatrième fréquence d'état et le quatrième nombre de messages d'état du mode de fonctionnement de validation de réception 14 peuvent être réduits.

De même, une fois qu'un message de validation d'ouverture est reçu, une surveillance fréquente n'est plus nécessaire, car l'ouverture du conteneur 2 est non seulement bien détectée par les moyens de détection, mais est par ailleurs validée par le destinataire final. Le dispositif de surveillance se trouve alors dans le mode de validation d'ouverture 13, et la troisième fréquence d'état et le troisième nombre de messages d'état du mode de fonctionnement de validation de réception peuvent encore être réduits.

Enfin, lorsqu'aucune ouverture n'est détectée, la surveillance est réduite au maximum. Le dispositif de surveillance se trouve alors dans le mode de fermeture 11, et la première fréquence d'état et le premier nombre de messages d'état du mode de fonctionnement de validation de réception sont très faibles.

Bien sûr, ceci est aussi valable pour les fréquences de supervision et le nombre de messages de supervision.

Or, comme on l'a dit plus tôt, lorsque le dispositif de surveillance n'est pas activé pour réaliser les fonctions de détection, de mesure, d'envoi de messages ou de réception de messages, le dispositif de surveillance se trouve dans le mode de veille 10 à basse consommation d'énergie.

Ainsi, en réduisant la fréquence et le nombre de messages lorsqu'une fréquence importante et un nombre de messages importants ne sont pas nécessaires, on réduit la consommation électrique du dispositif de surveillance et on augmente l'autonomie du dispositif de surveillance qui peut être de plusieurs années en utilisant une pile standard.

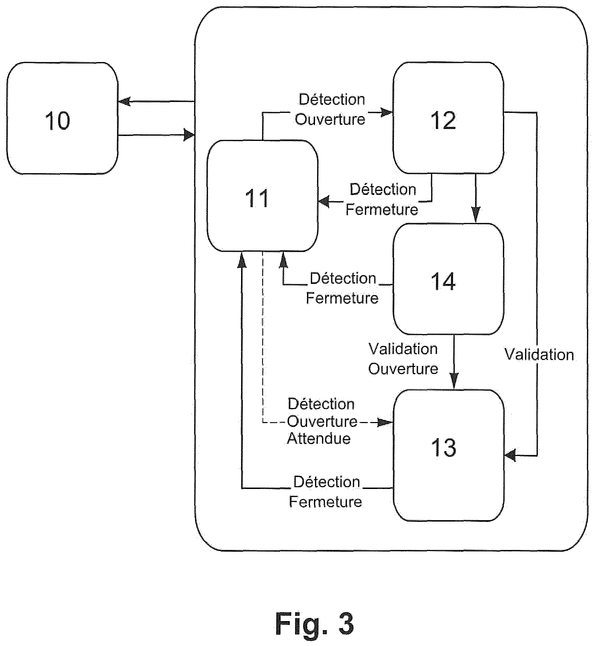

Le procédé de surveillance selon un deuxième mode de réalisation de l'invention est décrit en référence à la figure 3.

Le procédé de surveillance selon le deuxième mode de réalisation de l'invention ne diffère du premier mode de réalisation que par l'introduction du perfectionnement qui suit.

Dans le deuxième mode de réalisation, lorsque le dispositif de surveillance se trouve dans le mode de fonctionnement de fermeture 11, le module de communication « écoute » aussi le réseau LPWAN en activant la réception de l'émetteur-récepteur à une troisième fréquence de réception.

Le module de communication peut alors recevoir un message d'ouverture attendue provenant du destinataire final.

Le message d'ouverture attendue signifie tout d'abord que le destinataire final prévoit une ouverture du conteneur à court terme effectuée par une personne quelconque, par exemple par un opérateur. Le message d'ouverture attendue signifie de plus que le destinataire final autorise cette ouverture.

Lorsqu'un message d'ouverture attendue est reçu par le module de communication, le dispositif de surveillance est mis dans le mode de fonctionnement de validation d'ouverture 13.

L'opérateur peut alors ouvrir le conteneur et accéder à sa convenance à l'intérieur du conteneur.

On note que la troisième fréquence de réception est relativement faible, et correspond par exemple à une activation, une fois par jour, de la réception de l'émetteur-récepteur.

On note aussi que la troisième fréquence de réception est réglable, en fonction notamment de la probabilité qu'un message d'ouverture attendue soit reçu. La durée de l'activation de la réception est elle aussi réglable.

Bien entendu, l'invention n'est pas limitée aux modes de réalisation décrits mais englobe toute variante entrant dans le champ de l'invention telle que définie par les revendications.

Il est par exemple possible de prévoir que les modes de fonctionnement soient légèrement différents, par exemple que les messages de supervision soient différents, ou bien ne soient envoyés que dans certains modes de fonctionnement, ou bien qu'ils contiennent la capacité restante de la batterie, etc.

Bien que l'on ait uniquement précisé que la troisième fréquence de réception est réglable, toutes les fréquences évoquées ici sont réglables. De même, toutes les durées, au cours desquelles le dispositif de surveillance doit effectuer des fonctions de détection, de mesure, d'envoi de messages ou de réception de messages, sont réglables.

## Revendications

1. Procédé de surveillance de l'ouverture d'un contenant (2), le procédé de surveillance étant mis en œuvre par un dispositif de surveillance comprenant des moyens de détection de l'ouverture et de la fermeture du contenant (2) et un module de communication, le procédé de surveillance comprenant les étapes de :
- mettre le dispositif de surveillance dans un mode de fonctionnement de fermeture (11) lorsque les moyens de détection détectent que le contenant est fermé, dans lequel le module de communication envoie à une première fréquence d'état des messages de fermeture signifiant que les moyens de détection détectent que le contenant est fermé ;
- lorsque le dispositif de surveillance est dans le mode de fonctionnement de fermeture (11) et que les moyens de détection détectent que le contenant est ouvert, mettre le dispositif de surveillance dans un mode de fonctionnement d'alerte (12), dans lequel le module de communication envoie à une deuxième fréquence d'état des messages d'ouverture signifiant que les moyens de détection détectent que le contenant est ouvert ;
- lorsque le dispositif de surveillance est dans le mode de fonctionnement d'alerte (12) et que le module de communication reçoit un message de validation d'ouverture signifiant que l'ouverture du contenant a été validée, mettre le dispositif de surveillance dans un mode de fonctionnement de validation d'ouverture (13), dans lequel le module de communication envoie à une troisième fréquence d'état des messages d'ouverture signifiant que les moyens de détection détectent que le contenant est ouvert.

2. Procédé de surveillance selon la revendication 1, dans lequel la deuxième fréquence d'état est supérieure à la troisième fréquence d'état qui est supérieure à la première fréquence d'état.

3. Procédé de surveillance selon la revendication 1, dans lequel, lorsque le dispositif de surveillance est dans le mode de fonctionnement d'alerte (12) et que le module de communication reçoit un message d'accusé de réception signifiant qu'un message d'ouverture a bien été reçu, le dispositif de surveillance est mis dans un mode de fonctionnement de validation de réception (14) dans lequel le module de communication envoie à une quatrième fréquence d'état un message d'ouverture signifiant que les moyens de détection détectent que le contenant (2) est ouvert.

4. Procédé de surveillance selon la revendication 3, dans lequel, lorsque le dispositif de surveillance est dans le mode de fonctionnement de validation de réception (14) et que le module de communication reçoit le message de validation d'ouverture, le dispositif de surveillance est mis dans le mode de fonctionnement de validation d'ouverture (13).

5. Procédé de surveillance selon la revendication 3, dans lequel la quatrième fréquence d'état est inférieure à la deuxième fréquence d'état et est supérieure à la troisième fréquence d'état.

6. Procédé de surveillance selon la revendication 1, dans lequel, lorsque le dispositif de surveillance est dans le mode de fonctionnement de fermeture (11) et que le module de communication reçoit un message d'ouverture attendue signifiant qu'une ouverture du contenant est attendue et autorisée, le dispositif de surveillance est mis dans le mode de fonctionnement de validation d'ouverture (13).

7. Procédé de surveillance selon l'une des revendications précédentes, dans lequel un message de supervision du bon fonctionnement du dispositif de surveillance est envoyé à une première fréquence de supervision lorsque le dispositif est dans le mode de fonctionnement de fermeture (11), et/ou à une deuxième fréquence de supervision lorsque le dispositif de surveillance est dans le mode de fonctionnement d'alerte (12), et/ou à une troisième fréquence de supervision lorsque le dispositif de surveillance est dans le mode de fonctionnement de validation d'ouverture (13), et/ou à une quatrième fréquence de supervision lorsque le dispositif de surveillance est dans le mode de fonctionnement de validation de réception (14).

## Patentansprüche

1. Verfahren zur Überwachung des Öffnens eines Behälters (2), wobei das Verfahren zur Überwachung mittels einer Überwachungsvorrichtung durchgeführt wird, die Erfassungsmittel zum Erfassen des Öffnens und des Schließens des Behälters (2) und ein Kommunikationsmodul umfasst, wobei das Überwachungsverfahren die Schritte umfasst:
- Versetzen der Überwachungsvorrichtung in eine Schließbetriebsart (11), wenn die Erfassungsmittel erfassen, dass der Behälter geschlossen ist, in der das Kommunikationsmodul Schließnachrichten, die bedeuten, dass die Erfassungsmittel erfassen, dass der Behälter geschlossen ist, mit einer ersten Zustandsfrequenz sendet;
- wenn die Überwachungsvorrichtung in der Schließbetriebsart (11) ist und wenn die Erfassungsmittel erfassen, dass der Behälter geöffnet ist, Versetzen der Überwachungsvorrichtung in eine Alarmbetriebsart (12), in der das Kommunikationsmodul Öffnungsnachrichten, die bedeuten, dass die Erfassungsmittel erfassen, dass der Behälter geöffnet ist, mit einer zweiten Zustandsfrequenz sendet;
- wenn die Überwachungsvorrichtung in der Alarmbetriebsart (12) ist und wenn das Kommunikationsmodul eine Öffnungsvalidierungsnachricht empfängt, die bedeutet, dass das Öffnen des Behälters validiert wurde, Versetzen der Überwachungsvorrichtung in eine Betriebsart (13) der Validierung des Öffnens, in der das Kommunikationsmodul Öffnungsnachrichten, die bedeuten, dass die Erfassungsmittel erfassen, dass der Behälter geöffnet ist, mit einer dritten Zustandsfrequenz sendet.

2. Verfahren zur Überwachung nach Anspruch 1, bei dem die zweite Zustandsfrequenz größer als die dritte Zustandsfrequenz ist, die größer als die erste Zustandsfrequenz ist.

3. Verfahren zur Überwachung nach Anspruch 1, bei dem, wenn die Überwachungsvorrichtung in der Alarmbetriebsart (12) ist und wenn das Kommunikationsmodul eine Empfangsbestätigungsnachricht empfängt, die bedeutet, dass eine Öffnungsnachricht ordnungsgemäß empfangen wurde, die Überwachungsvorrichtung in eine Betriebsart (14) der Validierung des Empfangs versetzt wird, in der das Kommunikationsmodul eine Öffnungsnachricht, die bedeutet, dass die Erfassungsmittel erfassen, dass der Behälter (2) geöffnet ist, mit einer vierten Zustandsfrequenz sendet.

4. Verfahren zur Überwachung nach Anspruch 3, bei dem, wenn die Überwachungsvorrichtung in der Betriebsart (14) der Validierung des Empfangs ist und wenn das Kommunikationsmodul die Öffnungsvalidierungsnachricht empfängt, die Überwachungsvorrichtung in die Betriebsart (13) der Validierung des Öffnens versetzt wird.

5. Verfahren zur Überwachung nach Anspruch 3, bei dem die vierte Zustandsfrequenz kleiner als die zweite Zustandsfrequenz und größer als die dritte Zustandsfrequenz ist.

6. Verfahren zur Überwachung nach Anspruch 1, bei dem, wenn die Überwachungsvorrichtung in der Schließbetriebsart (11) ist und wenn das Kommunikationsmodul eine Nachricht einer erwarteten Öffnung empfängt, die bedeutet, das eine Öffnung des Behälters erwartet wird und autorisiert ist, die Überwachungsvorrichtung in die Betriebsart (13) der Validierung des Öffnens versetzt wird.

7. Verfahren zur Überwachung nach einem der vorhergehenden Ansprüche, bei dem eine Nachricht der Überwachung des ordnungsgemäßen Betriebs der Überwachungsvorrichtung mit einer ersten Überwachungsfrequenz gesendet wird, wenn die Vorrichtung in der Schließbetriebsart (11) ist, und/oder mit einer zweiten Überwachungsfrequenz, wenn die Überwachungsvorrichtung in der Alarmbetriebsart (12) ist, und/oder mit einer dritten Überwachungsfrequenz, wenn die Überwachungsvorrichtung in der Betriebsart (13) der Validierung des Öffnens ist, und/oder mit einer vierten Überwachungsfrequenz, wenn die Überwachungsvorrichtung in der Betriebsart (14) der Validierung des Empfangs ist.

## Claims

1. A monitoring method for monitoring the opening of a container (2), the monitoring method being performed by a monitoring device comprising means for detecting opening and closing of the container (2) and a communications module, the monitoring method comprising the steps of:
- putting the monitoring device into a closed mode of operation (11) when the detection means detect that the container is closed, in which mode the communications module sends closed messages at a first status repetition rate, the closed messages indicating that the detection means detect that the container is closed;
- when the monitoring device is in the closed mode of operation (11) and the detection means detect that the container is open, putting the monitoring device into a warning mode of operation (12) in which the communications module sends open messages at a second status repetition rate, the open messages indicating that the detection means detect that the container is open; and
- when the monitoring device is in the warning mode of operation (12) and the communications module receives an opening validated message indicating that opening of the container has been validated, putting the monitoring device in an opening validated mode of operation (13) in which the communications module sends open messages at a third status repetition rate, the open messages indicating that the detection means detect that the container is open.

2. A monitoring method according to claim 1, wherein the second status repetition rate is higher than the third status repetition rate, which is higher than the first status repetition rate.

3. A monitoring method according to claim 1, wherein, when the monitoring device is in the warning mode of operation (12), and the communications module receives an acknowledgment message indicating that an open message has been properly received, the monitoring device is put into a reception validated mode of operation (14) in which the communications module sends an open message at a fourth status repetition rate indicating that the detection means detect that the container (2) is open.

4. A monitoring method according to claim 3, wherein, when the monitoring device is in the reception validated mode of operation (14) and the communications module receives the opening validated message, the monitoring device is put into the opening validated mode of operation (13).

5. A monitoring method according to claim 3, wherein the fourth status repetition rate is lower than the second status repetition rate and higher than the third status repetition rate.

6. A monitoring method according to claim 1, wherein, when the monitoring device is in the closed mode of operation (11) and the communications module receives an opening expected message indicating that opening of the container is expected and authorized, the monitoring device is put into the opening validated mode of operation (13).

7. A monitoring method according to any preceding claim, wherein a supervision message relating to proper operation of the monitoring device is sent at a first supervision repetition rate when the device is in the closed mode of operation (11), and/or at a second supervision repetition rate when the monitoring device is in the warning mode of operation (12), and/or at a third supervision repetition rate when the monitoring device is in the opening validated mode of operation (13), and/or at a fourth supervision repetition rate when the monitoring device is in the reception validated mode of operation (14).
